Europäisches Patentamt

European Patent Office

Office européen des brevets

(19)

(11) **EP 1 291 078 A2**

(12) **EUROPÄISCHE PATENTANMELDUNG**

(43) Veröffentlichungstag:
12.03.2003 Patentblatt 2003/11

(51) Int Cl.⁷: **B01J 19/24**, C07C 201/06,
C07C 201/08, B01J 19/00

(21) Anmeldenummer: 02019065.8

(22) Anmeldetag: 28.08.2002

(84) Benannte Vertragsstaaten:
**AT BE BG CH CY CZ DE DK EE ES FI FR GB GR
IE IT LI LU MC NL PT SE SK TR**
Benannte Erstreckungsstaaten:
**AL LT LV MK RO SI**

(30) Priorität: **10.09.2001 DE 10144481
27.05.2002 DE 10223483**

(71) Anmelder: **BAYER AG
51368 Leverkusen (DE)**

(72) Erfinder:
• **Chrisochoou, Andreas, Dr.
51061 Köln (DE)**
• **Demuth, Ralf, Dr.
40724 Hilden (DE)**
• **Linn, Thomas, Dr.
41517 Grevenbroich (DE)**
• **Wagner, Paul, Dr.
40597 Düsseldorf (DE)**
• **Werner, Knud, Dr.
47800 Krefeld (DE)**

(54) **Rohrreaktor zur adiabatischen Nitrierung**

(57) Die Erfindung betrifft einen optimierten Rohrreaktor zur adiabatischen Mononitrierung von Aromaten, halogenierten Aromaten und halogenierten Kohlenwasserstoffen.

**Fig. 1**

**Beschreibung**

Die Erfindung betrifft einen optimierten Rohrreaktor zur adiabatischen Mononitrierung von Aromaten, halogenierten Aromaten und halogenierten Kohlenwasserstoffen.

Nitrierungen von Aromaten werden in zwei flüssigen Phasen durchgeführt. Die wässrige Phase enthält dabei Schwefelsäure als Katalysator und Salpetersäure als Reaktionspartner, ein weiterer Bestandteil kann beispielsweise Phosphorsäure sein, welche bei der Nitrierung von Toluol das Verhältnis der gebildeten Isomere beeinflusst. Die organische Phase enthält den zu nitrierenden Aromaten, zusätzlich enthält sie Teile des nitrierten Aromaten, der im Laufe der Reaktion entsteht.

Nitrierungen von Aromaten werden beispielsweise isotherm in Schlaufenreaktoren durchgeführt, wobei an einer oder an mehreren Stellen des Reaktors die wässrige Phase in der organischen Phase oder umgekehrt dispergiert wird. Die beiden Phasen werden im Schlaufenreaktor mehrmals umgepumpt, bevor sie den Reaktor verlassen. Dieser Umpumpstrom und sein Verhältnis zum Eingangsstrom legen fest, wie oft und in welchem zeitlichen Abstand die beiden Phasen die Dispergierstellen des Reaktors passieren.

Weiterhin ist es üblich, Nitrierungen von Aromaten adiabatisch durchzuführen. Durch diese Reaktionsführung erhitzt sich das Reaktionsgemisch mit zunehmendem Umsatz und auf Grund der Reaktionsbeschleunigung durch die erhöhte Temperatur wird die Reaktionszeit verkürzt. Ein weiterer Vorteil dieser Reaktionsführung ist, dass die hohe Temperatur des Reaktionsgemisches zum Abdampfen des bei der Reaktion entstehenden Wassers benutzt werden kann.

Üblicherweise wird eine adiabatische Nitrierung in einem Rohrreaktor durchgeführt. Hierbei hat man auf Grund der fehlenden Rück- oder Umpumpströmung einen gegenüber Schlaufenreaktoren vorteilhafteren Konzentrationsverlauf, der die Raumzeitausbeute in diesem Reaktortyp erhöht. Wegen des fehlenden Umpumpstroms müssen die Dispergierstellen hintereinander im Rohrreaktor angeordnet werden.

In EP 0 779 270 B1 ist ein Rohrreaktor beschrieben, welcher zur Herstellung einer aromatischen Mononitroverbindung eingesetzt werden kann. Der Rohrreaktor umfasst ein Rohr in dessen Innerem gewundene, flache Teile der Reihe nach derart angeordnet sind, dass ein vorderer Rand eines gewundenen flachen Teils im wesentlichen senkrecht zu einem rückwärtigen Rand des vorangegangenen Teils steht. Es befinden sich üblicherweise 50 oder weniger dieser gewundenen, flachen Teile in einem Reaktor, ihre bevorzugte Zahl wird mit 4 bis 12 angegeben. Nachteilig an diesem Reaktor ist, dass die darin angeordneten gebundenen, flachen Teile spezielle Formen aufweisen, die extra für diesen Reaktortyp angefertigt werden müssen.

In EP 0 489 211 B1 ist ein Jet-Aufprall-Reaktor zur Durchführung von Mononitrierungen beschrieben, welcher spezielle Einbauten enthält. Diese Einbauten bestehen aus Kugeln und Halbkugeln, welche mit Öffnungen versehen sind. Dieser Reaktor soll eine optimale Durchmischung von Flüssigphasen ermöglichen. Nachteilig an dem beschriebenen Reaktor ist, dass seine Konstruktion aufwändig ist und die beschriebenen Einbauten Sonderanfertigungen darstellen.

In DE 44 10 417 A1 und DE 44 11 064 A1 sind Verfahren zur adiabatischen Nitrierung von Toluolen bzw. Halogenbenzolen beschrieben. Die Nitrierreaktion wird dabei bevorzugt in einem Reaktor durchgeführt, welcher zur Dispergierung des Reaktionsgemisches Einbauten wie beispielsweise Lochbleche enthält. Die Zahl der Dipergierungsvorgänge sollte 2-50 betragen. Die genannten Offenlegungsschriften geben allerdings keinen Hinweis darauf, wie viele Einbauten im Reaktor vorhanden sein müssen und welche sonstigen zusätzlichen Bedingungen erfüllt sein müssen, um eine adiabatische Nitrierungsreaktion zum gewünschten Endumsatz zu führen.

Es bestand daher Bedarf, an einem einfach aufgebauten Rohrreaktor, der zur adiabatischen Herstellung von mononitrierten Verbindungen eingesetzt werden kann. Der Rohrreaktor sollte so konstruiert sein, dass die Dispergierwirkung ausreicht, um die Nitrierungsreaktion zum gewünschten Endumsatz zu führen.

Überraschenderweise wurde nun ein Rohrreaktor zur adiabatischen Mononitrierung von Aromaten, halogenierten Aromaten und halogenierten Kohlenwasserstoffen gefunden, welcher dadurch gekennzeichnet ist, dass er durch Böden mit Öffnungen in einer Mindestzahl von 4 bis 12 Kammern unterteilt ist, wobei durch die Böden ein Druckverlust von 0,5 bis 4 bar pro Boden erzeugt wird.

Untersuchungen haben gezeigt, dass bei einem Verfahren zur adiabatischen Mononitrierung der gewünschte Endumsatz von der Dispergierwirkung im Reaktor abhängt. Um eine ausreichend hohe Dispergierwirkung zu erhalten, werden in den erfindungsgemäßen Reaktor Böden mit geeigneten Öffnungen eingebaut, die den Reaktor in Kammern unterteilen. Durch die bei der adiabatischen Verfahrensführung auftretenden hohen Reaktionstemperaturen und die aggressiven Einsatzstoffe werden an das Material der Böden hohe Anforderungen gestellt. Vorzugsweise wird ein unter diesen Bedingungen inertes Material verwendet, besonders bevorzugt werden Tantalböden verwendet. Da dieses Material teuer ist, ist es für die Wirtschaftlichkeit des Nitrierverfahrens von besonderer Bedeutung, die Anzahl der Böden möglichst klein zu halten. Andererseits muss, um ein ideales Kolbenströmungsverhalten über den Reaktor zu gewährleisten, eine Mindestanzahl von Kammern und damit Böden vorhanden sein.

Der erfindungsgemäße Reaktor wird daher, durch Böden in 4 bis 12 Kammern, bevorzugt 6 bis 12 Kammern, besonders bevorzugt 7 bis 11 Kammern, eingeteilt. Die Böden fungieren als Dispergierelemente. Im erfindungsgemäßen Reaktor weisen die Böden Öffnungen auf. Bei den Öffnungen kann es sich um Schlitze, Löcher oder Bohrungen handeln. Besonders bevorzugt handelt es sich bei den Öffnungen um Bohrungen, da ihre Fertigung besonders einfach zu bewerkstelligen ist. Es können aber auch andere Öffnungsformen gewählt werden. Üblicherweise weist ein Boden für einen Massenstrom von 1 t/h 10 bis 25 Öffnungen, bevorzugt 15 bis 20 Öffnungen, auf.

Der erfindungsgemäße Reaktor weist vorzugsweise am unteren Ende mindestens eine Zufuhrmöglichkeit zum Einlass der Reaktanten auf und am oberen Ende mindestens eine Abfuhrmöglichkeit zur Entnahme des Reaktionsgemisches. In einer bevorzugten Ausführungsform des erfindungsgemäßen Reaktors weist dieser weiterhin Zufuhrmöglichkeiten für die organische und wässrige Phase auf, welche eine Zufuhr in die einzelnen im Reaktor befindlichen Kammern ermöglichen.

Neben Anzahl und Abstand der einzelnen Dispergierelemente ist für die Dispergierwirkung und damit für den gewünschten Endumsatz der Reaktion auch die Dispergierleistung von Bedeutung. Die Dispergierleistung muss in der Regel mechanisch in das Reaktionsgemisch eingebracht werden und sollte, um Betriebskosten zu senken, ebenfalls so klein wie möglich gewählt werden. Im Falle der erfindungsgemäß eingesetzten Böden mit Öffnungen wird die Dispergierwirkung in einem solchen Boden vom Druckabfall über diesen Boden bestimmt. Der Druckabfall bestimmt auf Grund von Festigkeitsgründen die Dicke der Böden und damit deren Preis.

Um über den ganzen Reaktor ein Kolbenströmungsverhalten zu erhalten und unerwünschte Rückströmungen durch die Böden zu vermeiden, werden im erfindungsgemäßen Reaktor Böden eingesetzt, welche einen Druckverlust von 0,5 bis 4 bar pro Boden erzeugen. Besonders bevorzugt werden zur adiabatischen Mononitrierung von Aromaten Böden eingesetzt, welche einen Druckverlust von 0,5 bis 3 bar, ganz besonders bevorzugt von 0,8 bis 2 bar, erzeugen.

Zur adiabatischen Mononitrierung von halogenierten Aromaten und halogenierten Kohlenwasserstoffen werden bevorzugt Böden eingesetzt, die einen Druckverlust von 0,5 bis 3 bar, ganz besonders bevorzugt von 0,5 bis 1,2 bar pro Boden, erzeugen.

Vorzugsweise wird der Druckverlust pro Boden möglichst gering gehalten, da zur Bereitstellung eines höheren Druckverlustes beispielsweise eine Pumpe höherer Leistung benötigt wird, die wiederum zu höheren Kosten des Gesamtverfahrens führt.

Erfindungsgemäß besonders bevorzugt ist es, die Zahl der Kammern und damit auch die Bodenzahl so niedrig wie möglich zu halten, da der Preis beispielsweise eines Tantalbodens im wesentlichen von der verwendeten Tantalmenge, d.h. der Dicke des Bodens abhängt. Muss man wegen der geringen Bodenzahl den Druckverlust pro Boden erhöhen, wirkt sich dies nicht so stark auf den Bodenpreis aus, da der Druckverlust nur mit seiner Quadratwurzel in die Bodendicke eingeht.

Zur Durchführung einer adiabatischen Mononitrierung im erfindungsgemäßen Rohrreaktor liegen die Reaktanten dabei in einem Zusammensetzungsbereich vor, wie er beispielsweise in US 5,313,009, in EP 0 436 443 B1 oder in DE 44 10 417 A1 beschrieben ist. Es sind aber auch andere Zusammensetzungen möglich.

Bei der Mononitrierung von halogenierten Aromaten liegen die Reaktanten in einem Zusammensetzungsbereich wie in US 4,453,027 oder DE 44 11 064 A1 beschrieben, vor. Auch hier sind andere Zusammensetzungen möglich.

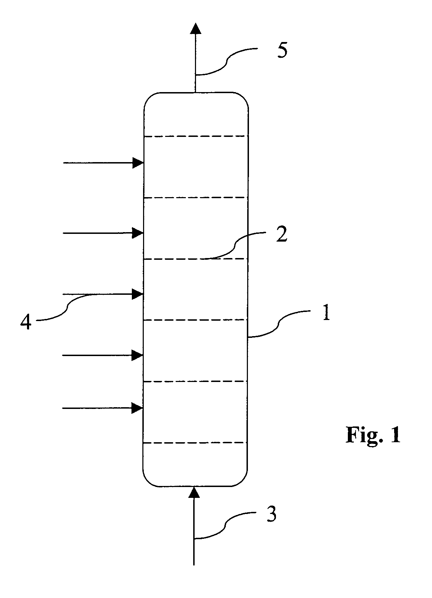

Eine besonders bevorzugte Ausführungsform des erfindungsgemäßen Reaktors ist in **Fig. 1** dargestellt. Es handelt sich dabei um einen Rohrreaktor **(1),** welcher durch mit Öffnungen versehene Bleche **(2)** in 7 Kammern unterteilt ist. Am unteren Ende befindet sich eine Zufuhrmöglichkeit für die Reaktanten **(3).** Über weitere Zufuhrmöglichkeiten **(4)** können die Reaktanten auch direkt in die einzelnen Kammern eingeleitet werden. Am oberen Ende des Reaktors befindet sich eine Abfuhrmöglichkeit **(5)** zum Auslass des Reaktionsgemisches.

## Beispiele

### Beispiel 1

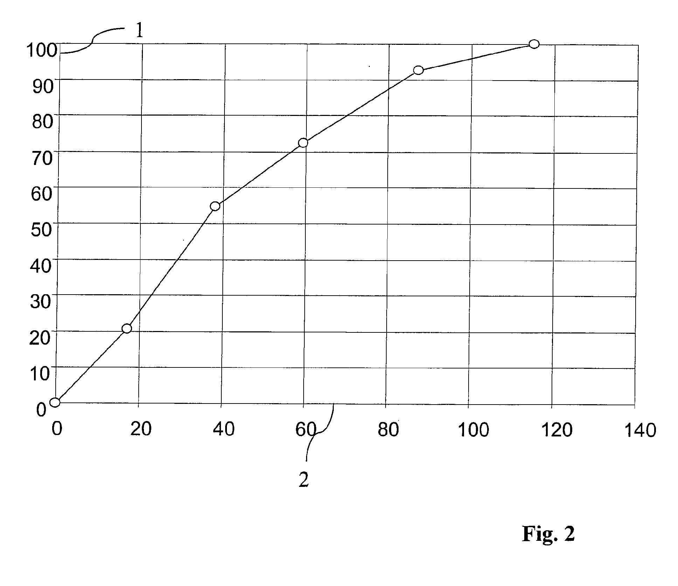

8 kg/h ca. 70 gew.-%ige Salpetersäure wurden 180 kg/h ca. 70 gew.-%iger Schwefelsäure zugeführt und bildeten eine 3 gew.-%ige Nitriersäure. Diese wurde in einem Wärmeübertrager auf ca. 80°C erwärmt. Anschließend wurden der Nitriersäure 9 kg/h Toluol in einem Dispergierelement zugemischt. Das Dispergierelement bestand aus Hastelloy und war, wie in DE 199 05 572 A1 dargestellt, ausgebildet. Der engste Strömungsquerschnitt auf der Säureseite betrug 7 mm$^2$, auf der Toluolseite 0,25 mm$^2$. Der Druckverlust auf der Säureseite betrug ca. 0,5 bar. Das Dispergierelement befand sich am Eingang zu einem wärmeisolierten Rohrreaktor (Durchmesser 50 mm, Höhe 3255 mm) aus emailliertem Stahl. Im Rohrreaktor befanden sich nahezu gleichmäßig über die gesamte Höhe verteilt weitere 18 Dispergierelemente aus Tantal, die als Scheiben einer Dicke von 1 mm ausgebildet und mit je 4 Bohrungen von 1,4 mm Durchmesser ausgestattet waren. Der Druckverlust pro Scheibe betrug ca. 0,5 bar. Am Reaktorende hatte sich die Temperatur auf 110°C erhöht und die gesamte Salpetersäure war umgesetzt. Der Umsatzverlauf wurde über die Temperaturerhöhung entlang der Reaktorachse bestimmt (s. Fig. 2). Die organische und die wässrige, säurehaltige Phase wurden in einem Gefäß bei 110°C getrennt. Die wässrige Phase wurde einem Verdampfer zugeführt, in dem das bei der Reaktion entstandene Wasser bei ca. 90°C entfernt wurde. Der dadurch wieder aufkonzentrierten Säure wurde, um die Anreicherung von Nebenprodukten zu verhindern, ein Teilstrom entnommen und durch frische Säure ersetzt. Anschließend wurde die Säure erneut mit Salpetersäure versetzt und dem Reaktor zugeführt.

In **Fig. 2** sind die Ergebnisse aus Beispiel 1 dargestellt, wobei der Umsatz in % **(1)** gegen die Verweilzeit in Sekunden **(2)** aufgetragen wurde.

### Beispiel 2

13 kg/h ca. 68 gew.-%ige Salpetersäure wurden 252 kg/h ca. 70 gew.-%iger Schwefelsäure zugeführt und bildeten eine 3 gew.-%ige Nitriersäure. Diese wurde in einem Wärmeübertrager auf ca. 80°C erwärmt. Anschließend werden der Nitriersäure 13,9 kg/h Toluol in einem Dispergierelement (analog zu Dispergierelement aus Beispiel 1) zugemischt. Das Dispergierelement befand sich am Eingang zu einem wärmeisolierten Rohrreaktor (Durchmesser 50 mm, Höhe 3255 mm) aus emailliertem Stahl. In diesem Rohrreaktor befanden sich 4 Dispergierelemente, die wie in Beispiel 1 beschrieben ausgebildet waren und sich auf einer Höhe von 200, 750, 1300 und 1800 mm im Reaktor befanden. Der Druckverlust pro Scheibe betrug aufgrund der gegenüber Beispiel 1 größeren Mengenströme ca. 1 bar. Am Reaktorende hatte sich die Temperatur auf 110°C erhöht und die gesamte Salpetersäure war umgesetzt. Die weitere Versuchsdurchführung sowie der weitere Versuchsaufbau war analog zu Beispiel 1.

### Beispiel 3

8,3 kg/h ca. 68 gew.-%ige Salpetersäure wurden 180 kg/h ca. 70 gew.-%-iger Schwefelsäure zugeführt und bildeten eine 3 gew.-%ige Nitriersäure. Diese wurde in einem Wärmeübertrager auf ca. 80°C erwärmt. Anschließend wurden der Nitriersäure 8,1 kg/h Toluol in einem zu Beispiel 1 analogen Dispergierelement zugemischt. Das Dispergierelement befand sich am Eingang zu einem wärmeisolierten Rohrreaktor (Durchmesser 50 mm, Höhe 3255 mm) aus emailliertem Stahl. Im Rohrreaktor befanden sich 6 Dispergierelemente, die als 1 mm dicke Scheiben ausgebildet waren und mit je 3 Bohrungen von 1,36 mm Durchmesser ausgestattet waren. Die Dispergierelemente befanden sich im Reaktor in einer Höhe von ca. 200, 500, 750, 1000, 1300 und 1800 mm. Der Druckverlust pro Scheibe betrug ca. 1 bar. Am Reaktorende hatte sich die Temperatur auf 110°C erhöht und die gesamte Salpetersäure war umgesetzt. Die weitere Versuchsdurchführung sowie der weitere Versuchsaufbau war analog zu Beispiel 1.

### Beispiel 4

Folgende Einsatzmengen wurden für die anschließende Berechnung zugrunde gelegt: 10 t/h Toluol; 9,6 t/h 68 gew.-%ige Salpetersäure; 208 t/h 70 gew.-%ige Schwefelsäure.

Wählt man den Bohrungsdurchmesser d wie in Beispiel 1 zu 1,4 mm und hält den Massenstrom M durch den Bohrungsquerschnitt Q konstant, so erhält man jeweils denselben Druckverlust im Boden wie in den Beispielen 1 bis 3. Für Beispiel 1 ergibt sich:

$$M_{Beispiel\ 4} / Q_{Beispiel\ 4} = M_{Beispiel\ 1} / Q_{Beispiel\ 1} \qquad \text{(Gleichung 1)}$$

Q ergibt sich aus dem Bohrungsdurchmesser d und der Bohrungszahl B zu:

$$Q = \pi/4 \cdot d^2 \cdot B \qquad \text{(Gleichung 2)}$$

Setzt man Gleichung 2 in Gleichung 1 ein und löst nach $B_{Beispiel\ 4}$ auf, so ergibt sich:

$$B_{Beispiel\ 4} = B_{Beispiel\ 1} \cdot d^2_{Beispiel\ 1}/d^2_{Beispiel\ 4} \cdot M_{Beispiel\ 4}/M_{Beispiel\ 1} \qquad \text{(Gleichung 3}$$

Man berechnet nun folgende Bohrungszahlen:

Für Beispiel 1:

$$B = 4 \cdot 1,4^2/1,4^2 \cdot (10000+9600+208000)/(8+180+9) = 4621\ \text{Bohrungen/Boden}$$

Für Beispiel 2:

$$B = 4 \cdot 1,4^2/1,4^2 \cdot (10000+9600+208000)/(13+252+13,9) = 3264 \text{ Bohrungen/Boden}$$

Für Beispiel 3:

$$B = 3 \cdot 1,36^2/1,4^2 \cdot (10000+9600+208000)/(8,3+180+8,1) = 3281 \text{ Bohrungen/Boden}$$

Wählt man nun einen Reaktorquerschnitt mit einem Durchmesser D = 700 mm und ordnet die Bohrungen in Dreiecksteilung an, kann man den Bohrungsabstand t folgendermaßen berechnen:

Bei einer Dreiecksteilung, wie z.B. in DIN 28182 beschrieben, befinden sich die Bohrungsmittelpunkte auf den Spitzen eines gleichseitigen Dreiecks der Seitenlänge t. Da alle Winkel in einem solchen Dreieck gleich sind und 60° betragen, befindet sich von jeder Bohrung nur 1/6 auf dem Dreieck. Fasst man die auf dem Dreieck liegende Bohrungsfläche zusammen, ergibt sich 3 · 1/6 = ½ der Bohrungsfläche je Dreieck. Die Anzahl Bohrungen B ist daher gleich der halben Anzahl gleichseitiger Dreiecke, in die man den Reaktorquerschnitt aufteilen kann. Die Anzahl der Dreiecke berechnet sich aus dem Verhältnis des Reaktorquerschnitts zur Fläche eines Dreiecks zu:

$$\pi/4 \cdot D^2 / \sqrt{3}/4 \cdot t^2 = \pi/\sqrt{3} \cdot D2/t2 \qquad \text{(Gleichung 4)}$$

Damit ergibt sich:

$$B = \tfrac{1}{2} \cdot \pi/\sqrt{3} \cdot D^2/t^2 \qquad \text{(Gleichung 5)}$$

Aufgelöst nach t:

$$t = D \; \sqrt{[\pi/(2B3)]} \qquad \text{(Gleichung 6)}$$

Damit ergeben sich folgende Bohrungsabstände:

$$\text{Für Beispiel 1: } t = 700 \cdot \sqrt{[\pi(2 \cdot 4621\sqrt{3})]} = 9,8 \text{ mm}$$

Für Beispiel 2 und 3 ergeben sich dann analog 11,7 mm und 11,6 mm.

Als Bodenmaterial wird Tantal ES gemäß VdTÜV-Werkstoffblatt 382 gewählt. Nach diesem Blatt beträgt die 0,2% Dehnungsgrenze $R_{p0,2}$ = 94 N/mm$^2$ bei 130 °C.

Zur Berechnung der erforderlichen Bodendicke wird aus AD-Merkblatt B5 Formel 19 herangezogen. Der Sicherheitsbeiwert wird zu S = 1,5 angesetzt, der Berechnungsbeiwert zu C = 0,4. Vernachlässigt man die Zuschläge $c_1$ und $c_2$, ergeben sich die in der Tabelle angegebenen Bodendicken.

| Beispiel Nr. | Überdruck | Verschwächungs- beiwert | Bodendicke | Bodenzahl | Tantal- menge |
|---|---|---|---|---|---|
| 1 | 0,5 bar | 0,86 | 8,5 mm | 18 | 977 kg |
| 2 | 1 bar | 0,88 | 11,9 mm | 4 | 304 kg |
| 3 | 1 bar | 0,88 | 11,9 mm | 6 | 456 kg |

**Patentansprüche**

1.  Rohrreaktor zur adiabatischen Mononitrierung von Aromaten, halogenierten Aromaten und halogenierten Kohlenwasserstoffen, **dadurch gekennzeichnet, dass** der Rohrreaktor durch Böden mit Öffnungen in 4 bis 12 Kammern unterteilt wird, wobei durch die Böden ein Druckverlust von 0,5 bis 4 bar pro Boden erzeugt wird.

2. Rohrreaktor gemäß Anspruch 1 zur adiabatischen Mononitrierung von Benzol oder Toluol, **dadurch gekennzeichnet, dass** durch die Böden ein Druckverlust von 0,8 bis 2 bar pro Boden erzeugt wird.

3. Rohrreaktor gemäß Anspruch 1 zur adiabatischen Mononitrierung von Chlorbenzol oder ortho-Dichlorbenzol **dadurch gekennzeichnet, dass** durch die Böden ein Druckverlust von 0,5 bis 1,2 bar pro Boden erzeugt wird.

4. Rohrreaktor nach einem oder mehreren der vorangegangenen Ansprüche 1 bis 3, **dadurch gekennzeichnet, dass** es sich bei den Öffnungen um Bohrungen handelt.

5. Rohrreaktor gemäß einem oder mehreren der vorangegangenen Ansprüche 1 bis 4, **dadurch gekennzeichnet, dass** es sich bei den Böden um Tantalböden handelt.

6. Rohrreaktor nach einem oder mehreren der vorangegangenen Ansprüche 1 bis 5, **dadurch gekennzeichnet, dass** er Zufuhrmöglichkeiten für die organische und wässrige Phase aufweist, welche eine Zufuhr in die einzelnen im Reaktor befindlichen Kammern ermöglichen.

Fig. 1

**Fig. 2**